# EUROPÄISCHE PATENTANMELDUNG

(11) **EP 4 706 741 A1**
(43) Veröffentlichungstag der Anmeldung: **11.03.2026**
(21) Anmeldenummer: 25199427.3
(22) Anmeldetag: 01.09.2025
(51) Int. Cl.: A61M 39/26, A61F 9/00

(54) **CHIRURGISCHES SYSTEM FÜR OPHTHALMOLOGISCHE EINGRIFFE**

(30) Priorität: 06.09.2024 DE 102024208499
(71) Anmelder: Geuder AG, 69126 Heidelberg (DE)
(72) Erfinder: Eichhorn, Frank, 74934 Reichartshausen (DE); Schimmer, Lea, 69181 Leimen (DE)
(74) Vertreter: Ullrich & Naumann PartG mbB

(57) **Zusammenfassung**

Ein chirurgisches System für ophthalmologische Eingriffe, umfassend ein Gehäuse mit darin befindlichen Funktionseinheiten sowie mit Bedien- und Anzeigeelementen sowie Anschlüssen für ophthalmologische Geräte, wobei die Anschlüsse einen Luftanschluss umfassen, ist dadurch gekennzeichnet, dass der Luftanschluss selbstschließend ausgeführt ist, damit bei nicht oder nicht korrekt angeschlossenem Luftschlauch keine Druckluft in den Bereich des sterilen Umfelds des Systems gelangt.

## Beschreibung

Die Erfindung betrifft ein chirurgisches System für ophthalmologische Eingriffe, umfassend ein Gehäuse mit darin befindlichen Funktionseinheiten sowie mit Bedien- und Anzeigeelementen sowie Anschlüssen für ophthalmologische Geräte, wobei die Anschlüsse einen Luftanschluss umfassen.

Chirurgische Systeme der hier in Rede stehenden Art sind seit Jahren aus der Praxis bekannt. In Bezug auf die integrierten Funktionseinheiten sowie in Bezug auf die grundsätzliche Funktion sei lediglich beispielhaft auf die DE 10 2017 215 677 B3, DE 10 2015 003 799 A1 und US 2015/0250939 A1 verwiesen. Aus diesen Druckschriften sind, jeweils für sich gesehen, gattungsbildende ophthalmologische Systeme bekannt.

Chirurgische Systeme der hier in Rede stehenden Art sind seit Jahren aus der Praxis bekannt. Beispielsweise ein zu einem Vitreo-Cutter führender Druckluftschlauch wird am Gehäuse über Luer-Anschlüsse angeschlossen. Bei dem sogenannten Luer-Lock-System erfolgt der Anschluss durch Einstecken des Schlauchendes in eine entsprechende Luer-Box. Der dichte Verschluss wird durch teilweises Drehen des Anschlussstücks generiert.

In der Praxis kann es passieren, dass der Druckluftschlauch nicht oder nicht richtig angeschlossen ist. Gibt der Operateur die Druckluft frei, besteht aufgrund eines nicht erfolgten Anschlusses der Druckluftleitung die Gefahr, dass der Operationsraum durch die nicht sterile Druckluft kontaminiert wird. Dies gilt es zu vermeiden.

Der Erfindung liegt daher die Aufgabe zugrunde, einen sicheren Umgang mit der Druckluft zu gewährleisten, insbesondere eine Kontamination des Operationsraums mit Druckluft wirksam zu verhindern. Außerdem soll sich das System von wettbewerblichen Systemen unterscheiden.

Voranstehende Aufgabe ist durch die Merkmale des Anspruchs 1 gelöst. Danach umfasst der Luftanschluss, genauer gesagt der Anschluss für Druckluft, beispielsweise zur Versorgung eines Druckluft benötigenden Instruments, ein selbstschließendes System. Dieses System ist derart ausgelegt, dass bei nicht oder nicht korrekt angeschlossenen Luftschlauch bzw. bei nicht eingestecktem Luftschlauchanschluss keine Druckluft nach außerhalb gelangt, insbesondere nicht in den Bereich des sterilen Umfelds des Systems im Operationsraum.

In vorteilhafter Weise und insbesondere zur Realisierung einer einfachen Ausführung ist der selbstschließende Luftanschluss zumindest ganz überwiegend oder insgesamt mechanisch ausgeführt. Im Konkreten kann der selbstschließende Luftanschluss feder- und/oder magnetunterstützt sein. Beim ordnungsgemäßen Einstecken zum Beispiel eines Schlauchanschlusses eines Vitreo-Cutters wird der öffnet. An Luftanschluss freigegeben. Es ist wesentlich, dass die Mechanik des selbstschließenden Luftanschlusses im Wesentlichen innerhalb des Gehäuses angeordnet ist, wobei außerhalb des Gehäuses eine Einsteckbuchse, zum Einstecken, Eindrehen oder Einschrauben eines Schlauchanschlusses, zumindest teilweise positioniert ist.

Bei dem Anschluss kann es sich um einen modifizierten selbstdichtenden Luer-Lock-Anschluss handeln, entsprechend den voranstehenden Ausführungen mit einem integrierten Rückschlagventil, welches durch ordnungsgemäßes Einstecken eines Schlauchanschlusses, und nur dadurch, geöffnet wird.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung eines bevorzugten Ausführungsbeispiels der Erfindung anhand der Zeichnung zu verweisen. In Verbindung mit der Erläuterung eines bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen die
- Fig. 1: in einer schematischen Ansicht den Aufbau eines selbstschließenden Luftanschlusses, wie er bei dem beanspruchten chirurgischen System Anwendung finden kann,
- Fig. 2: in einer schematischen Ansicht den selbstschließenden Luftanschluss im gesperrten Zustand, und
- Fig. 3: in einer schematischen Ansicht den selbstschließenden Luftanschluss im kontaktierten, geöffneten Zustand.

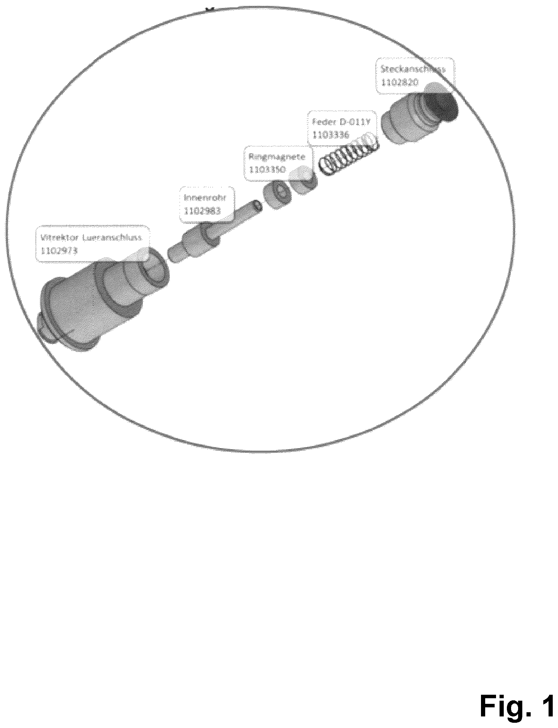

Fig. 1 zeigt links unten den Vitrektor-Luer-Anschluss, wobei am äußeren Ende die nach außerhalb des Gehäuses ragende Anschlussbuchs zu sehen ist. Ein sich nach rechts anschließender Ringflansch liegt am Gehäuse an. Die Anschlussbuchse erstreckt sich nach innerhalb des Gehäuses. Ihr folgt ein Innenrohr. Das Rückschlagventil wird durch Ringmagnete und eine Feder unterstützt. Rechts danach befindet sich ein Steckanschluss.

Durch Einstecken des Schlauchanschlusses wird das so gebildete Rückschlagventil entgegen die Federkraft geöffnet, nämlich dann, wenn der Schlauchanschluss ordnungsgemäß eingesteckt ist. Eine wirksame mechanische Realisierung des selbstschließenden Luftanschlusses, mit integralem Rückschlagventil, ist geschaffen.

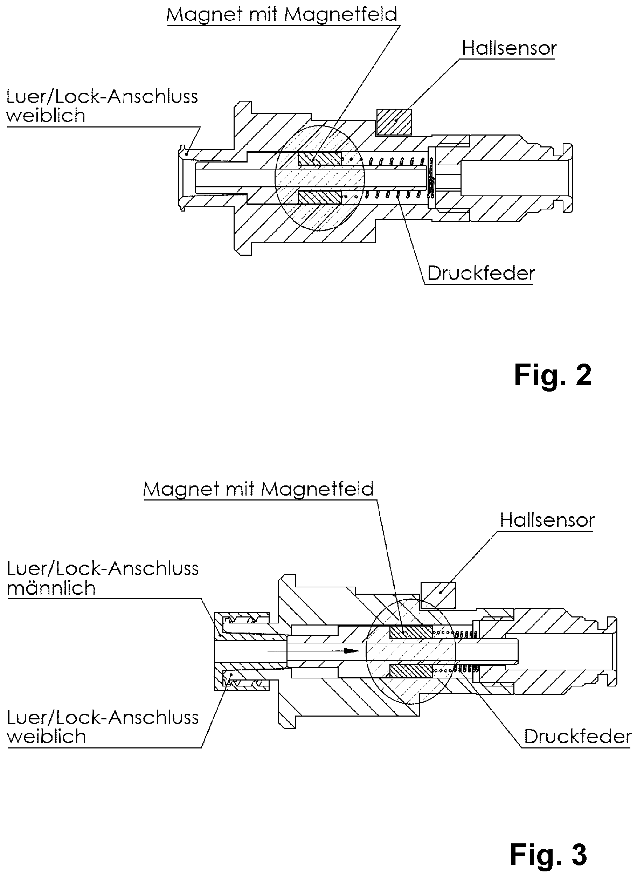

Unter Bezugnahme auf die Figuren 2 und 3 sei die Funktion wie folgt erklärt:
Wenn Zubehör, beispielsweise der Anschluss eines Cutters, ins Gerät eingesteckt wird, werden Ringmagnete auf einem Röhrchen in Richtung Geräteinnenseite geschoben und lösen dort einen in der Nähe angebrachten Hall-Sensor aus. Dadurch erkennt die Software des Gerätes, dass Zubehör angeschlossen ist und gibt die Funktion frei. Ohne - ordnungsgemäß - angeschlossenes Zubehör ist die Funktion gesperrt und es kann keine Luft in das sterile OP-Umfeld strömen.

Hinsichtlich weiterer vorteilhafter Ausgestaltungen der erfindungsgemäßen Lehre wird zur Vermeidung von Wiederholungen auf den allgemeinen Teil der Beschreibung sowie auf die beigefügten Ansprüche verwiesen.

Schließlich sei ausdrücklich darauf hingewiesen, dass das voranstehend beschriebene Ausführungsbeispiel der erfindungsgemäßen Lehre lediglich zur Erörterung der beanspruchten Lehre dient, diese jedoch nicht auf das Ausführungsbeispiel einschränkt.

## Patentansprüche

1. Chirurgisches System für ophthalmologische Eingriffe, umfassend ein Gehäuse mit darin befindlichen Funktionseinheiten sowie mit Bedien- und Anzeigeelementen sowie Anschlüssen für ophthalmologische Geräte, wobei die Anschlüsse einen Luftanschluss umfassen,
**dadurch gekennzeichnet, dass** der Luftanschluss selbstschließend ausgeführt ist, damit bei nicht oder nicht korrekt angeschlossenem Luftschlauch keine Druckluft in den Bereich des sterilen Umfelds des Systems gelangt.

2. System nach Anspruch 1, **dadurch gekennzeichnet, dass** der selbstschließende Luftanschluss zuminderst ganz überwiegend oder insgesamt mechanisch ausgeführt ist.

3. System nach Anspruch 2, **dadurch gekennzeichnet, dass** der selbstschließende Luftanschluss ein feder- und/oder magnetunterstütztes Rückschlagventil umfasst, welches beim ordnungsgemäßen Einstecken z.B. des Schlauchanschlusses eines Vitreo-Cutters öffnet.

4. System nach einem der Ansprüche 1 bis 3, **dadurch gekennzeichnet, dass** die Mechanik des selbstschließenden Luftanschlusses innerhalb des Gehäuses angeordnet ist, wobei außerhalb des Gehäuses eine Einsteckbuchse, zum Einstecken, Eindrehen oder Einschrauben eines Schlauchanschlusses positioniert ist.

5. System nach einem der Ansprüche 1 bis 4, **dadurch gekennzeichnet, dass** der Anschluss als modifizierter selbstdichtender Luer-Lock-Anschluss ausgeführt ist.
